# EUROPÄISCHE PATENTSCHRIFT

(11) **EP 3 702 340 B1**
(45) Veröffentlichungstag und Bekanntmachung des Hinweises auf die Patenterteilung: **14.05.2025**
(21) Anmeldenummer: 20161611.7
(22) Anmeldetag: 08.12.2017
(51) Int. Cl.: C04B 111/28

(54) **FORMTEIL UMFASSEND EINE CHEMISCH GEBUNDENE PHOSPHATKERAMIK**
SHAPED ARTICLE COMPRISING A CHEMICALLY BONDED PHOSPHATE CERAMIC
PIÈCE MOULÉE COMPRENANT UNE CÉRAMIQUE PHOSPHATIQUE LIÉE CHIMIQUEMENT

(30) Priorität: 08.12.2016 DE 102016123842
(43) Veröffentlichungstag der Anmeldung: 02.09.2020
(62) Teilanmeldung aus: 17835807.3
(73) Patentinhaber: IBV Holding GmbH, 49078 Osnabrück (DE)
(72) Erfinder: Breker, Ronald, 41836 Hückelhoven (DE)
(74) Vertreter: Abitz & Partner

(56) Entgegenhaltungen:
- EP-A1- 0 078 507
- EP-A1- 1 571 239
- EP-B1- 0 861 216
- DE-T2- 69 515 634
- DE-T2- 69 515 634
- ANONYMOUS: "Vubonite quick start", SYMBION: VUBONITE QUICK START ENGLISH, 1 March 2005 (2005-03-01), pages 1 - 4, XP093035123

## Beschreibung

Formteil umfassend eine chemisch gebundene Phosphatkeramik.

Sogenannte chemisch gebundene Phosphatkeramiken werden für eine Vielzahl von Anwendungen eingesetzt, beispielsweise für prothetische Anwendungen, als Dentalzemente, sowie bei der Herstellung von strukturellen Bauteilen. Ein wichtiges Anwendungsgebiet dabei ist die Herstellung von nicht brennbaren Bauteilen, die im Brandfall möglichst wenig bzw. keine gesundheitsschädlichen Emissionen abgeben. Beispielsweise kann es im Falle eines Brandes bei Bauteilen, die aus Phenolharzen und/oder modifizierten Kunststoffen wie schwer entflammbaren ungesättigten Polyesterharzen und schwer entflammbaren Epoxidharzen hergestellt wurden, zur Emission von gesundheitsschädlichen Gasen kommen. Diese können beispielsweise Blausäure, Kohlenmonoxid, Stickoxide, Schwefeldioxid, Fluorwasserstoff, Kohlendioxid sowie Bromwasserstoff sein.

Aus der Druckschrift EP 0 861 216 B1 ist eine chemisch gebundene Phosphatkeramik bekannt, die unter dem Markennamen Vubonite^{®} vertrieben wird. Die Fertigung von Formteilen aus Vubonite^{®} führt häufig zu einem geometrischen Verzug der Formteile bedingt durch Schrumpf. Dieser geometrische Verzug und die Spannungen im Formteil sind die Ursache von Haarrissen auf der Oberfläche. Weiterhin ist eine Oberflächenveredelung der Formteile z. B. durch eine dekorative Nassapplikation wie Lackieren nicht möglich, da es aufgrund der Oberflächenspannungen zu Adhäsionsbrüchen zwischen der Oberfläche des Formteils und dem Applikationsaufbau kommt.

Weiterhin erlaubt Vubonite^{®} nicht die gezielte Einstellung der Viskosität und weist darüber hinaus eine hohe Massendichte von ungefähr 1750 kg/m³ auf. Mit Vubonite^{®} gefertigte Formteile erreichen weiterhin lediglich eine thermische Isolation (Wärmeleitfähigkeit) von ungefähr 1000 mW/ (m·K).

Nachstehend wird eine chemisch gebundene Phosphatkeramik beschrieben, die gegenüber den oben genannten Nachteilen verbessert ist. Weiterhin wird eine Zusammensetzung zur Herstellung der chemisch gebundenen Phosphatkeramik, die Phosphatkeramik, sowie Verfahren zur Herstellung der Phosphatkeramik und Verfahren zur Herstellung von Formteilen offenbart. Ziel der vorliegenden Erfindung ist es, Formteile aus der Zusammensetzung bereitzustellen.

Gegenstand der Erfindung ist das im unabhängigen Anspruch 1 definierte Formteil. Dieses Formteil umfasst eine chemisch gebundene Phosphatkeramik, wobei die Phosphatkeramik zwischen 0,2 Massen% bis 2 Massen% Al, zwischen 0,2 Massen% bis 1,5 Massen% Zn und zwischen 20 bis 35 Massen% CaO enthält.

### Beschrieben wird eine:

Zusammensetzung zur Herstellung einer chemisch gebundenen Phosphatkeramik mit:
Komponente A) als flüssige Komponente aufweisend 50 bis 100 Gewichtsprozent einer phosphorsauren Verbindung in wässriger Lösung und
Komponente B) ein Calcium-Silikat,
   wobei das Mischungsverhältnis der Komponente A) zu Komponente B) 45:70 bis 110:140, beziehungsweise 45:100 bis 100:140 Gewichtsanteile beträgt.

Aufgrund des hohen Anteils der phosphorsauren Verbindung, die in wässriger Lösung vorliegt, kann es besonders gut zu einer Polykondensation zwischen dem Phosphat als Komponente A) und dem Calcium-Silikat als Komponente B) kommen, sodass eine chemisch gebundene Phosphatkeramik gebildet werden kann.

Unter einer "chemisch gebundenen Phosphatkeramik" im Sinne der hier beschriebenen technischen Lehre werden feste, keramikähnliche Materialien verstanden, die mittels einer exothermen, energieabgebenden Polykondensation zwischen Phosphat-Bestandteilen und Calcium-Silikat-Bestandteilen von Mischungen gebildet werden können. Das Aushärten der chemisch gebundenen Phosphatkeramiken geschieht dabei über die exotherme Polykondensationsreaktion und benötigt keinen, für andere keramische Materialien üblichen Sinterschritt bei hohen Temperaturen. Während des Sinterns wird aus körnigen oder pulvrigen Grünmaterialien bei Temperaturen von häufig größer 800 °C bis zu 2500 °C ein keramisches Gefüge gebildet.

Die Zusammensetzung zur Herstellung der chemisch gebundenen Phosphatkeramik ist besonders gut geeignet für Herstellungsverfahren von Formteilen, wie Pressen und/oder Spritzgießen, bei denen in die Kavität eines Formwerkzeugs die Mischung der Komponente A) und B) der Zusammensetzung eingebracht wird und anschließend das Press-Formteil durch hohe Pressdrücke größer als 10 daN/cm² und durch Temperaturen bis zu 160 °C innerhalb weniger Minuten ausgehärtet werden kann. Bei der Spritztechnologie wird die chemisch gebundene Phosphatkeramik mit bis zu 3000 bar in die Kavität gefördert. Weitere Fertigungstechnologien sind Faserspritzen, Vakuuminjektion und das Pultrudieren von Endlosprofilen.

Unter "Formteilen" im Sinne der vorliegenden Erfindung werden alle Teile verstanden, die mit der hier beschriebenen chemisch gebundenen Phosphatkeramik gefertigt werden können, also z. B. 2-dimensionale Platten sowie 3-dimensionale Teile, wie fertige Bauteile und Halbzeuge und auch Laminate, bei denen Schichten, enthaltend die Phosphatkeramik mit Schichten anderer Zusammensetzung, wie z. B. Mineralwollschichten, Metallfaserschichten oder Metallschichten kombiniert werden. Bei den Schichten kann es sich insbesondere um Lagen handeln, die durch Tränken von Fasermatten mit der Zusammensetzung für die chemisch gebundene Phosphatkeramik erhalten werden können. Durch Übereinanderstapeln der getränkten Fasermatten im Rahmen von z. B. Handauflegeverfahren kann die Dicke der Lagenaufbauten variiert werden.

Die Komponente A) weist neben Wasser keine weiteren Bestandteile auf. Durch Variation der Anteile an Wasser kann die Viskosität der Zusammensetzung für unterschiedliche Spritzgieß- und/oder Pressverfahren sowie Laminierverfahren angepasst werden. Bevorzugt beträgt der Anteil der phosphorsauren Verbindung an der Komponente A) 60 bis 100 Gew% für Anwendungen wie Pressen, Spritzgießen und/oder Laminierverfahren, wie z. B. Handauflegeverfahren, und der restliche Anteil Wasser zur Einstellung der Viskosität. Bei Verfahren wie Pressen und Spritzgießen beträgt dabei bevorzugt der Anteil der Komponente A) zur Komponente B) 45:70 bis 110:100 45:100 bis 100:100 Gewichtsanteile und bei Verfahren, bei denen ein Formteil gegossen wird oder zusätzlich noch Schäumungsmittel eingesetzt werden, beträgt der Anteil der Komponente A) zu B) 100:140 Gewichtsanteile.

Weiterhin beträgt der Anteil an der phosphorsauren Verbindung in wässriger Lösung an der Komponente A) bevorzugt 70 Gew% bis 95 Gew%, wobei dann 5 bis 30 Gew% Wasser sind.

Die phosphorsaure Verbindung in wässriger Lösung kann insbesondere eine wässrige Lösung von Phosphorsäure umfassen oder daraus bestehen. Generell kann bei allen Zusammensetzungen zur Herstellung einer chemisch gebundenen Phosphatkeramik 85%ige beziehungsweise 95%ige Phosphorsäure als phosphorsaure Verbindung in wässriger Lösung eingesetzt werden. 95%-ige Phosphorsäure weist gegenüber der niedrigeren Konzentration von 85% einen deutlich geringeren Wasseranteil auf, der bei Pressteilen vorteilhaft ist, da der Anteil von freiem Wasser im Formteil niedriger ist und somit die elektrische Leitfähigkeit (Bemessungsisolationsspannung nach DIN EN 60670-12 und DIN EN 61439) im Formteil erheblich reduziert ist.

Eine weitere Variante einer hier beschriebenen Zusammensetzung weist in der Komponente A) als flüssige Komponente oder als Kombination einer flüssigen Komponente mit Feststoffen zusätzlich noch zumindest eine der folgenden Verbindungen auf:
- 0,2 bis 10, bevorzugt 0,2 bis 7 Gewichts% einer Aluminium-Verbindung,
- 0,2 bis 10, bevorzugt 0,2 bis 9 Gewichts% einer Zink-Verbindung, und
- eine Bor-Sauerstoff-Verbindung.

Dabei ist es möglich, dass nur eine der oben genannten Verbindungen, zumindest zwei oder auch alle drei Verbindungen in den hier beschriebenen Zusammensetzungen vorhanden sind. Aufgrund der zusätzlichen Verbindungen eignen sich derartige Zusammensetzungen neben den Spritzgieß-Verfahren und Pressen auch für Handauflegeverfahren, bei denen beispielsweise Faserschichten oder Faserlagen, wie zum Beispiel Fasermatten aus Naturfasern oder Mineralfasern in eine Form eingebracht werden, mit den hier beschriebenen Zusammensetzungen getränkt werden und anschließend weitere Faserschichten aufgebracht und wiederum getränkt werden können, bis eine gewünschte Schichtdicke erreicht wird. Beispielsweise können in Tränkungsanlagen mehrere Fasermatten gleichzeitig mit den hier beschriebenen Zusammensetzungen in einem Verfahrensschritt getränkt werden.

Die zusätzlichen Verbindungen können eine kontrolliert exotherme Polykondensationsreaktion zwischen den Phosphat-Bestandteilen und den Calcium-Silikat-Bestandteilen der hier beschriebenen Zusammensetzungen bewirken, wobei die Zink-Verbindung und die Aluminium-Verbindung als Aktivatoren dienen und die Polykondensation beschleunigen, während die Bor-Sauerstoffverbindung als Retarder dient und die Polykondensation verzögert, sodass eine verlängerte Topfzeit (Verarbeitungszeit) resultiert.

Diese Verbindungen können entweder der flüssigen Komponente A) zugegeben werden und in dieser gelöst werden, bevor und/oder während das Abmischen der Komponente A) mit der Komponente B) vorgenommen wird. Weiterhin können alle Verbindungen beziehungsweise ein Teil der Verbindungen als Feststoffe zugegeben werden, nachdem die flüssige Komponente A) mit der festen Komponente B) gemischt wurde.

Das Zusetzen der Verbindungen während des Mischens und/oder nach dem Mischen der Komponenten A) und B) ergibt den Vorteil, dass die Mischzeit für die Beimengung der Feststoffe gegenüber einem kompletten Lösen der Feststoffe in der Komponente A) vor dem Abmischen mit der Komponente B) verringert werden kann. Beispielsweise kann die Mischzeit beim Einbringen der Feststoffe in die Mischung der Komponenten A) und B) wenige Minuten betragen, während das Lösen der Feststoffe in der flüssigen Komponente A) mehrere Stunden in Anspruch nehmen kann. Beispielsweise können bestimmte Aluminium-Verbindungen nur schwer oder nicht in Lösung zu bringen sein, sodass die phosphorsaure wässrige Komponente A) über mehrere Stunden zusätzlich auf erhöhte Temperatur, beispielsweise rund 40 °C, aufgeheizt werden muss.

Weiterhin kann die Reaktivität der Mischung der Komponenten A) und B) auch dadurch gesteuert werden, dass die oben genannten Verbindungen nach dem Mischen der Komponenten A) und B) oder während des Mischens beider Komponenten als Feststoffe zugesetzt werden. Dies ist dann von besonderem Vorteil, wenn Umwelteinflüsse wie Raumtemperaturveränderungen und/oder Luftfeuchtigkeitsveränderungen auftreten, auf die schnell reagiert werden muss.

Weiterhin kann die Komponente A) 0,2 bis 9 Gew%, bevorzugt 1 bis 9 Gew%, weiter bevorzugt 2 bis 4 Gew% der Bor-Sauerstoff-Verbindung enthalten.

Höhere Anteile der Bor-Sauerstoff-Verbindung als Retarder erhöhen die Topfzeit der hier beschriebenen Zusammensetzungen und ermöglichen damit eine längere Verarbeitungszeit.

Als Bor-Sauerstoff-Verbindung können vor allem Borate, z. B. Borsäure, aber bevorzugt Borax Na₂B₄O₇×10H₂0, eingesetzt werden.

Weiterhin kann der Anteil der Aluminium-Verbindung in der Komponente A) 0,2 bis 7 Gew%, bevorzugt 2 bis 6 Gew%, betragen.

Die Aluminium-Verbindung als Aktivator führt mit steigenden Konzentrationen zu einer zunehmend exothermen Polykondensation zwischen dem Phosphat-Bestandteil der Komponente A) und dem Calcium-Silikat-Bestandteil der Komponente B), wobei aber auch die Aluminium-Verbindung, beispielsweise durch die Bildung von Aluminium-Phosphaten an der Polykondensation beteiligt ist.

Die Aluminium-Verbindung kann insbesondere Oxid- und/oder Hydroxid-Verbindungen des Aluminiums wie Aluminiumoxid Al₂O₃ und/oder Aluminiumhydroxid Al(OH)₃ umfassen, bevorzugt Aluminiumhydroxid, weiter bevorzugt amorphes Aluminiumhydroxid.

Als Aluminium-Verbindungen sind generell Verbindungen bevorzugt, die praktisch unlöslich sind in Wasser und lediglich Löslichkeiten von nicht größer als 2 mg/l, bevorzugt 1,5 mg/l aufweisen. Eine bevorzugte Aluminium-Verbindung ist Aluminiumhydroxid, wobei sich besonders gut amorphes Aluminiumhydroxid dazu eignet, vor dem Abmischen mit der Komponente B) in der flüssigen Komponente A) gelöst zu werden, um ein Ausflocken von Aluminium-Verbindungen während des Lösungsprozesses zu vermindern oder zu vermeiden.

Der Anteil der Zink-Verbindung an der Komponente A) kann 0,2 bis 9 Gew%, bevorzugt 1 bis 6 Gew%, weiter bevorzugt 1 bis 3 Gew%, betragen. Dabei führen höhere Gewichtsprozentanteile an der Zink-Verbindung als Aktivator zu einer stärker exothermen Polykondensationsreaktion, wobei auch die Zink-Verbindung, beispielsweise durch die Bildung von Zink-Phosphaten, an der Polykondensation beteiligt sein kann.

Als Zink-Verbindung lassen sich besonders gut Oxid- und/oder Hydroxid-Verbindungen des Zinks einsetzen, wie z. B. bevorzugt Zinkoxid ZnO und/oder Zinkhydroxid Zn(OH)₂.

Generell sollten in Wasser nichtlösliche Zink-Verbindungen eingesetzt werden, da diese besonders die Polykondensation ermöglichen, ähnlich zu den nichtlöslichen Aluminium-Verbindungen, wie oben bereits diskutiert. Die nichtlöslichen Zink-Verbindungen sollten eine Löslichkeit von nicht größer 2 mg/l in Wasser aufweisen.

Der Anteil der phosphorsauren Verbindung in wässriger Lösung an der Komponente A) kann insbesondere 50 bis 75 Gew%, bevorzugt 55 bis 68 Gew%, betragen. Derartige Gewichtsprozentanteile sind besonders gut geeignet für Zusammensetzungen, bei denen mittels eines Handauflegeverfahrens mit Faserschichten Formteile beliebiger Dicke durch Tränken der Faserschichten mit den hier beschriebenen Zusammensetzungen hergestellt werden können. Nach der Härtung der Zusammensetzung zu der chemisch gebundenen Phosphatkeramik liegen dann die Fasermatten eingebettet in der chemischen Phosphatkeramik als Matrix vor.

Beim Handauflegeverfahren sind die oben genannten Anteile der phosphorsauren Verbindung an der Komponente A) besonders geeignet, eine kontrollierte exotherme Polykondensation zu ermöglichen, bei der insbesondere die Temperaturerhöhung während der exothermen Polykondensation nicht mehr als 20 °C/m² Oberfläche hergestellter chemisch gebundener Phosphatkeramik beziehungsweise je m² Oberfläche hergestellter Laminatanordnung mit der chemisch gebundenen Phosphatkeramik beträgt.

Das Mischungsverhältnis der Komponente A) zu Komponente B) kann weiterhin bevorzugt 75:70 bis 110:100, beziehungsweise 70:100 bis 100:100, bevorzugt 75:100 bis 100:100 bzw. 70:100 bis 90:100 Gewichtsanteile betragen. Insbesondere für das Pressen werden Gewichtsanteile der Komponente A) zu B) von 70:100 bis 90:100 eingestellt. Für das Handauflegeverfahren sind Verhältnisse von 75:70 bis 110:100, beziehungsweise 75:100 bis 100:100 Gewichtsanteilen bevorzugt.

Gemäß einer weiteren Ausführungsform der hier beschriebenen Zusammensetzung kann die Komponente A) zusätzlich eine Metall-Sulfat-Verbindung enthalten, die ebenfalls wieder der flüssigen Komponente A) zugemischt und gelöst werden kann, bevor die Abmischung mit der Komponente B) erfolgt oder die während bzw. nach der Mischung beider Komponenten A) und B) diesen als Feststoff zugesetzt werden kann.

Der Anteil der Metall-Sulfat-Verbindung an der Komponente A) kann dabei 0,01 bis 1 Gew% betragen, wobei diese Metall-Sulfat-Verbindung bevorzugt ausgewählt ist aus den Sulfaten der Alkalimetalle oder Kombinationen davon, insbesondere Na, z. B. Na₂SO₄ oder K₂SO₄. Die Metall-Sulfat-Verbindung hat gegenüber der Zink-Verbindung oder der Aluminium-Verbindung eine geringere Wirkung auf die Exothermie der exothermen Polykondensation, wodurch die Reaktionsgeschwindigkeit gezielter beeinflusst werden kann.

Die Komponente A) kann weiterhin zusätzlich eine MetallHalogenid-Verbindung enthalten, deren Anteil an der Komponente A) 0,01 bis 1 Gew% betragen kann. Als MetallHalogenid-Verbindung können insbesondere NaBr oder NaCl eingesetzt werden, wobei aber auch Kalium oder CäsiumVerbindungen der Bromide oder Chloride möglich sind.

Gemäß einer weiteren Ausführungsform der vorliegenden Zusammensetzung kann das Calcium-Silikat der Komponente B) ausgewählt sein aus Wollastonit, insbesondere β-Wollastonit.

Wollastonit ist ein natürlich vorkommendes Mineral der chemischen Zusammensetzung Ca₃[Si₃O₉]. Seine Kristallstruktur weist SiO₃²⁻-Ketten auf, die über Calciumionen verknüpft sind, so dass ein Einfachkettensilikat gebildet wird, das Teil der Gruppe der Inosilikate ist. Der Erweichungspunkt von β-Wollastonit liegt oberhalb von 1500 °C und erlaubt die Herstellung von feuerfesten Formteilen.

Das Calcium-Silikat beziehungsweise das Wollastonit kann gemäß einer weiteren Ausführungsform der vorliegenden Zusammensetzung eine Korngröße D₅₀ von < 70 µm, bevorzugt 5µm - <50 µm, insbesondere 5 bis 25 µm, aufweisen.

Der Erfinder hat erkannt, dass bei ansonsten gleicher Zusammensetzung der Komponente A) und gleichbleibender Rührgeschwindigkeit beim Mischen der Komponenten A) und B) die Temperaturerhöhung der exothermen Reaktion dadurch gesteuert werden kann, dass Calcium-Silikate mit verschiedenen Korngrößen als Komponente B) zugemischt werden. Dabei nimmt die Exothermie der Polykondensationsreaktion mit kleiner werdender Korngröße D₅₀ der Calcium-Silikat-Partikel zu. Ein weiterer Vorteil besteht darin, dass homogene und spannungsfreie Oberflächen realisiert werden können.

Auch geschäumte Formteile können mit hier beschriebenen n Zusammensetzungen hergestellt werden. Die Korngröße D₅₀ des Calcium-Silikats, insbesondere des Wollastonits, ist unter anderem bestimmend für die Struktur der in der geschäumten chemisch gebundenen Phosphatkeramik erzeugten Zellen mit Gaseinschlüssen, wobei durch den Zusatz von den unten beschriebenen Schäumungsmitteln die Größe dieser Zellstrukturen beeinflusst werden kann.

Zur Herstellung von geschäumten chemisch gebundenen Phosphatkeramiken mit reduzierten Dichten kann die Komponente A) weiterhin ein Schäumungsmittel, bevorzugt Wasserstoffperoxid H₂O₂ und/oder mit Kohlensäure H₂CO₃ versetztes Wasser, enthalten.

Das Zusetzen der Schäumungsmittel erlaubt ein Aufschäumen der chemisch gebundenen Phosphatkeramik durch das Freisetzen von Sauerstoff im Falle von Wasserstoffperoxid beziehungsweise Kohlendioxid bei Kohlensäure, wobei die Dichte der chemisch gebundenen Phosphatkeramik von Werten im ungeschäumten Zustand von etwa 1750 kg/m³ je nach Anteil an Schäumungsmittel auf bis unter 300 kg/m³ reduziert werden kann. Dazu können die Schäumungsmittel in einem Anteil von bis zu 40 Gew% an der Komponente A) hinzugegeben werden. Eine Gewichtsersparnis ist besonders entscheidend bei Formteilen, die ein niedriges Gewicht aufweisen müssen, wie z. B. Bauteile im Schiffsbau oder im Flugzeugbau. Wasserstoffperoxid kann insbesondere in einer Konzentration von bis zu 50 Gew% hinzugegeben werden.

Zur Herstellung von Brandschutzteilen für Decken, Wände und Böden, beispielsweise im Schiffbau, Flugzeugbau, Schienenfahrzeugbau, Hochbau und Tunnelbau, werden auch Zusammensetzungen gesucht, die halogenfrei sind, um beispielsweise die Entwicklung von HBr oder HCl im Brandfall zu unterbinden. Deshalb ist es weiterhin vorteilhaft, dass bestimmte hier beschriebene Zusammensetzungen, die zur Herstellung einer halogenfreien, nicht brennbaren chemisch gebundenen Phosphatkeramik dienen, frei sind von Halogenverbindungen. Dabei sind insbesondere beide Komponenten A) und B) gemäß einer weiteren Ausführungsform halogenfrei. Zur Herstellung von feuerfesten Formteilen weist die hier beschriebene Zusammensetzung gemäß einer weiteren Ausführungsform auch keine organischen Verbindungen auf. Dies ermöglicht es besonders gut, feuerfeste anorganische chemisch gebundene Phosphatkeramiken herzustellen, die bei erhöhten Temperaturen bis zu 1500°C und größer temperaturbeständig sind.

Gemäß einer weiteren Ausführungsform der hier beschriebenen Zusammensetzung besteht diese ausschließlich aus den Komponenten A) und B) und weist somit keine anderen Komponenten auf. Dies bedeutet, dass abgesehen von den oben beschriebenen Verbindungen der Komponenten A) und B) keine weiteren Verbindungen in hier beschriebenen Zusammensetzungen vorhanden sind. Beispielsweise können somit hier beschriebene Zusammensetzungen anders als beispielsweise aus dem Stand der Technik bekannte Phosphatkeramiken keine Zirkon-Verbindungen, beispielsweise ZrO₂, und auch keine Eisenoxidverbindungen enthalten. Dies erlaubt eine technisch weniger aufwändige und kostengünstigere Herstellung von hier beschriebenen Zusammensetzungen. Dabei ist aber zu beachten, dass selbst bei keiner gezielten Zugabe von Eisenoxidverbindungen zu den Komponenten A) und B) der Zusammensetzung die ausgehärtete hier beschriebenen Phosphatkeramik dennoch geringste Mengen an Eisenoxid in der Größenordnung von 0,1 bis 0,4 Massen% enthalten kann, da dieses Oxid als Verunreinigung beispielsweise im Wollastonit enthalten sein kann.

Es wird auch ein Verfahren zur Herstellung einer chemisch gebundenen Phosphatkeramik unter Verwendung einer der oben genannten Zusammensetzungen mit den Verfahrensschritten beschrieben:
A) Mischen der Komponenten A) und B),
B) Härtung der Mischung der Komponenten A) und B) unter Bildung der chemisch gebundenen Phosphatkeramik.

Bevorzugt wird im Verfahrensschritt A) die Komponente B) als Feststoffkomponente eines Calcium-Silikats in die flüssige Komponente A) eingebracht. Dabei ist es vorteilhaft, wenn die Mischung gerührt ist, wobei die Mischzeiten zwischen einer Minute bis kleiner 10 Minuten, bevorzugt 2,5 bis 3 Minuten, betragen können und die Rührgeschwindigkeit 500 bis 5000 rpm, bevorzugt 1000 bis 3000 rpm, weiter bevorzugt 2000 bis 3000 rpm, beträgt.

Im Verfahrensschritt A) können dabei nach dem Mischen der Komponente B) mit der flüssigen Komponente A) weitere feste Bestandteile der Komponente A) in die Mischung eingebracht werden, beispielsweise die bereits oben beschriebenen Verbindungen, die Aluminium-Verbindung, die Zink-Verbindung, die Metall-Sulfat-Verbindung oder die Bor-Sauerstoff-Verbindung. Dies hat den Vorteil, dass beim Zusetzen der festen Bestandteile der Komponente A) während des Mischens der Komponenten A) und B) oder nach deren Mischen eine kürzere Mischzeit resultieren kann. Alternativ ist es auch möglich, alle als Feststoffe vorliegenden Komponenten zuerst in der phosphorsauren flüssigen Verbindung der Komponente A) zu lösen und anschließend die Abmischung mit der Komponente B) durchzuführen.

Bei einer weiteren Variante eines beschriebenen Verfahrens zur Herstellung einer chemisch gebundenen Phosphatkeramik werden während des Verfahrensschrittes A) nach dem Mischen der Komponenten A) und B) Verbindungen hinzugefügt, die ausgewählt sind aus:
- Schäumungsmitteln,
- Fasern
- Polyesterpasten mit ungesättigtem Polyesterharz,
- Wasser,
- Füllstoffen, oder
- Kombinationen dieser Verbindungen.

Schäumungsmittel, bevorzugt Wasserstoffperoxid und/oder Kohlensäure, beispielsweise in der Form von kohlensäurehaltigem Wasser, können bevorzugt in einem Anteil von 2 bis 10 Gew% bezogen auf die Gesamtmischung A) und B) zugesetzt werden, um wie bereits oben beschrieben eine Schäumung der chemisch gebundenen Phosphatkeramik zu erreichen.

Bei Zugabe von Schäumungsmitteln nach Mischen der Komponenten A) und B) ergibt sich in Summe eine höhere Rührzeit und damit eine reduzierte Verarbeitungszeit (Topfzeit). Um eine reduzierte Topfzeit zu verhindern, kann wie bereits oben beschrieben das Schäumungsmittel auch direkt der flüssigen Komponente A) zugesetzt werden. Um die Wirkung von Wasserstoffperoxid als Schäumungsmittel zu verstärken, empfiehlt es sich, dessen Zugabe in zeitlich versetzten Schritten durchzuführen.

Als Fasern können bevorzugt Mineralfasern und Naturfasern eingesetzt werden, wobei als Naturfasern z. B. Baumwollfasern, Hanffasern, Flachsfasern, Sisalfasern, Vulkanfasern und Thermoplastfasern oder auch Kombinationen dieser genannten Fasern eingesetzt werden. Vulkanfasern können beispielsweise auf der Basis von Zellstoff-Fasern, die mit Zinkchlorid behandelt wurden, hergestellt werden. Sisalfasern werden aus den Fasern der Blätter einiger Agavenarten gewonnen.

Als Mineralfasern können beispielsweise Glasfasern, Carbonfasern, Basaltfasern oder Steinwollfasern sowie eine Kombination dieser Fasern eingesetzt werden. Weiterhin können auch Mineralfasern und Naturfasern kombiniert werden. Die Fasern werden bevorzugt in einem Anteil von bis zu 70 Vol%, etwa 2 bis 30 Gew% an der Gesamtmischung der Komponenten A) und B) hinzugegeben. Auch Metallfasern können eingesetzt werden. Die Fasern erlauben eine Verstärkung von Formteilen, also gezielte strukturelle Festigkeiten von Formteilen und die Erhaltung der Integrität der Bauteilkonstruktion über Normen entsprechenden lange Zeiträume im Hochtemperaturbereich, wobei die Fasern in die chemisch gebundene Phosphatkeramik als Matrixmaterial eingelagert werden.

Polyesterpasten mit ungesättigtem Polyesterharz können bevorzugt in einem Styrol-Lösungsmittel vorliegen und einen Anteil von weniger als 65 Gew% Farbpigmenten aufweisen. Vor allen Dingen bei hier beschriebenen geschäumten chemisch gebundenen Phosphatkeramiken kann der Zusatz der Polyesterpaste in einem Anteil von bis zu 3 Gew%, bevorzugt 0,3 bis 2,2 Gew%, eine verbesserte Zellstruktur und eine farbliche Variation des geschäumten Materials bedingen.

Zur Anpassung der Viskosität der Mischung der Komponenten A) und B) kann auch je nach Bedarf noch Wasser in einem Anteil von bevorzugt 2 bis 15 Gew% an der Gesamtmischung A) und B) hinzugegeben werden.

Füllstoffe können beispielsweise ausgewählt werden aus Materialien wie Flugasche, Glashohlkörpern, Blähglasgranulaten, expandiertem Polystyrol und Sand und können in einem Anteil von weniger als 30 Gew%, bevorzugt 20 Gew%, an der Mischung A) und B) hinzugegeben werden.

Die Rührzeiten bei dem zusätzlichen Einrühren dieser genannten Verbindungen in die Mischung der Komponenten A) und B) können bis zu 10 Minuten, bevorzugt ein bis drei Minuten, betragen.

Bei einer weiteren Variante eines hier beschriebenen Verfahrens läuft nach der Mischung der Komponenten A) und B) eine exotherme Reaktion unter Bildung der chemisch gebundenen Phosphatkeramik ab, wobei diese exotherme Reaktion kontrolliert ist und die Temperatur während der exothermen Reaktion um nicht mehr als 20 °C, bevorzugt nicht mehr als 17 °C und weiter bevorzugt nicht mehr als 12 °C, pro m² Oberfläche der hergestellten chemisch gebundenen Phosphatkeramik ansteigt.

Wie bereits weiter oben beschrieben läuft bei Mischung der Komponenten A) und B) eine exotherme Polykondensation zumindest zwischen der Phosphatkomponente und der Calcium-Silikat-Komponente ab, wobei gegebenenfalls auch noch vorhandene weitere Verbindungen, wie die Zink-Verbindung oder die Aluminium-Verbindung, die Metall-Sulfat-Verbindung sowie die Bor-Sauerstoff-Verbindung an der Polykondensation partizipieren können. Bei Einstellung der Gewichtsprozentanteile der Komponente A) und Anpassen der Korngröße D₅₀ der Calcium-Silikat-Feststoffpartikel der Komponente B) kann eine kontrollierte chemische Polykondensation ablaufen, bei der insbesondere ohne Kühlung die Temperatur um nicht mehr als 20 °C ansteigt. Eine derartige kontrollierte exotherme Polykondensationsreaktion ist besonders vorteilhaft bei den bereits oben beschriebenen Handauflegeverfahren, bei denen Fasermatten oder Faserschichten mit einer Mischung der Komponenten A) und B) getränkt werden und anschließend diese Mischungen aushärten, wobei je nach Anzahl der Faserschichten spannungsfreie und formstabile Formteile mit unterschiedlicher Dicke hergestellt werden können.

Es wurde festgestellt, dass durch Erhöhung des Gewichtsprozentanteils der phosphorsauren Verbindung in wässriger Lösung in der Komponente A) die Temperaturerhöhung (Exothermie) der Polykondensationsreaktion stärker abläuft. Ebenso ist bei Erhöhung der Gewichtsprozentanteile der Zink-Verbindung und der Aluminium-Verbindung in der Komponente A) eine erhöhte Wärmeentwicklung während der Polykondensation zu beobachten. Dieser Effekt beider Verbindungen ist aber im Vergleich zu der phosphorsauren Verbindung nicht so stark ausgeprägt und somit ermöglichen diese Verbindungen sowie die weiteren bereits beschriebenen Feststoffe eine Feineinstellung der kontrolliert exothermen Polykondensationsreaktion.

Eine Erhöhung des Anteils der Bor-Sauerstoff-Verbindung, bevorzugt des Borax, als Retarder führt in erster Linie zu einer erhöhten Topfzeit.

Wie bereits oben beschrieben kann bei gleicher Zusammensetzung der Komponente A) und gleichbleibender Rührzeit sowie Rührgeschwindigkeit die exotherme Reaktion der Polykondensation auch dadurch verstärkt werden, dass die Korngröße D₅₀ der Calcium-Silikat-Partikel vermindert wird.

Aufgrund der kontrollierten exothermen Reaktion können formgetreue, spannungsfreie Formteile hergestellt werden, wobei diese Formteile keinen Schrumpf oder keine Oberflächenspannungen aufweisen. Dieser Schrumpf und die Oberflächenspannungen erzeugen Adhäsionsbrüche zu einem Applikationswerkstoff, z. B. einem Lack, und weisen Haarrisse in der Oberflächenmatrix auf.

Zur Durchführung einer kontrollierten exothermen Polykondensation enthält die Komponente A) bevorzugt:
- 50 bis 75 Gew%, bevorzugt 55 bis 68 Gew%, der phosphorsauren Lösung, und -falls vorhanden-
- 0,2 bis 9 Gew%, bevorzugt 1,5 bis 5 Gew%, der Zink-Verbindung,
- 0,2 bis 7 Gew%, bevorzugt 4 bis 6 Gew%, der Aluminium-Verbindung und
- 0,2 bis 9 Gew%, bevorzugt 2 bis 9 Gew%, der Borsauerstoffverbindung,
- wobei das Mischungsverhältnis mit der Komponente B), dem Calciumsilikat, 75:70 bis 110:100, weiterhin 70:100 bis 100:100 Gewichtsanteile beträgt.

Sollte auch die Metall-Sulfat-Verbindung vorhanden sein, so wird diese in einem Anteil von 0,2 bis 4 Gew% an der Komponente A) eingesetzt, um eine kontrollierte exotherme Polykondensation zu ermöglichen.

Eine Variation der Gewichtsanteile dieser Verbindungen innerhalb dieser Grenzen ermöglicht besonders einfach ein Einstellen der kontrollierten exothermen Reaktion. Prinzipiell lässt sich aber auch eine kontrollierte exotherme Polykondensation dadurch einstellen, dass die oben genannten Verbindungen der Komponente A) in größeren Gewichtsprozentanteilen variiert werden, wie weiter oben beschrieben.

Des Weiteren beschrieben ist ein Verfahren zum Herstellen einer zweidimensionalen oder dreidimensionalen Laminatanordnung unter Verwendung einer der oben beschriebenen Zusammensetzungen mit den Verfahrensschritten:
A1) Mischen der Komponente A) und B),
B1) Aufbringen der Mischung der Komponenten A) und B) auf die zumindest eine erste Faserschicht, und
C1) Aushärten der Mischung der Komponenten A) und B) unter Bildung des Bauteils mit der chemisch gebundenen Phosphatkeramik und der zumindest einen ersten Faserschicht.

Ein derartiges Verfahren ist besonders gut dazu geeignet, als sogenanntes Handauflegeverfahren zur Herstellung von Laminaten zu dienen, bei denen eine erste Faserschicht in eine Matrix aus der chemisch gebundenen Phosphatkeramik eingebettet ist. Besonders vorteilhaft werden Mischungen der Komponenten A) und B) eingesetzt, die eine kontrollierte exotherme Reaktion wie oben beschrieben ermöglichen, so dass auch mittels des Handauflegeverfahrens besonders einfach formgetreue, spannungsfreie Formteile mit homogenen (spannungsfreien) Oberflächen und Laminatanordnungen hergestellt werden können.

Die zwei- oder dreidimensionale Laminatanordnung kann beispielsweise als zweidimensionales Laminat eine Platte sein und als 3D-Laminat ein Bauteil oder ein Halbzeug.

Eine Variation des gerade genannten Verfahrens unter Verwendung einer zweiten Faserschicht umfasst die zusätzlichen Verfahrensschritte:
B2) die zweite Faserschicht wird auf die erste Faserschicht nach dem Verfahrensschritt B1) aufgebracht,
B3) die Mischung der Komponenten A) und B) wird auf die zumindest zweite Faserschicht aufgebracht,
   - wobei im Verfahrensschritt C1) die Mischung der Komponenten A) und B) unter Bildung des Bauteiles umfassend eine chemisch gebundene Phosphatkeramik mit zumindest der ersten und zweiten Faserschicht ausgehärtet wird.

Durch ein derartiges Verfahren kann zumindest eine zweite Faserschicht auf der ersten Faserschicht angeordnet werden und so die Dicke der herzustellenden Laminatanordnung bestimmt werden. Mittels des hier beschriebenen Verfahrens können beliebige weitere Faserschichten auf bereits getränkten Schichten angeordnet werden und anschließend durch Aushärten getrocknet werden. Diese Methode des Handauflegens kann auch dafür eingesetzt werden, feuerwiderstandsfähige Formteile herzustellen, die geringe Wärmeleitfähigkeiten bis unter 110 mW/ (m·K) aufweisen können und bei denen eine gezielte Delamination stattfinden kann (siehe genauere Beschreibung dieser Bauteile weiter unten).

Die Faserschichten können insbesondere Mineralfaserschichten oder Naturfaserschichten sein.

Weiterhin kann bei dem genannten Herstellungsverfahren im Verfahrensschritt C1) die Mischung der Komponenten A) und B) einer Mikrowellenstrahlung ausgesetzt werden.

Die Mikrowellenstrahlung bedingt, dass die Formteile innerhalb sehr kurzer Zeit, in der Regel 10 bis 30 Sekunden, trocknen, wobei sich eine sehr gute Oberflächenqualität ohne Oberflächenspannungen ergibt.

Die Mikrowellenstrahlung kann dabei bevorzugt Strahlung mit einer Energiedichte von 4 bis 60 kW/m³ aufweisen. Im Wesentlichen können dabei bevorzugt zwei verschiedene Frequenzen, zum einen eine Frequenz von 915 MHz mit einer Leistung von weniger als 125 kW und zum anderen eine Strahlung mit einer Frequenz von 2,45 GHz mit einer Leistung von < 30 kW, verwendet werden.

Der hier beschriebenene Einsatz von Mikrowellenstrahlung kann sowohl bei Formteilen angewendet werden, bei denen lediglich die chemisch gebundene Phosphatkeramik, eventuell zusammen mit darin eingebetteten Fasern oder Faserschichten, vorhanden ist, sie kann aber auch eingesetzt werden, wenn Schichten umfassend die chemisch gebundene Phosphatkeramik auf Mineralwollschichten angeordnet werden.

Unter "Mineralwollschichten" werden dabei allgemein Schichten aus mineralischen Fasern verstanden. Mineralwollschichten können z. B. Glaswollschichten oder Steinwollschichten sein. Steinwollschichten können beispielsweise aus Rohstoffen wie Spat, Dolomit, Basalt, Diabas, Anorthosit sowie Recyclingmaterial hergestellt werden.

Bei der Anwesenheit von Mineralwollschichten führt die Anwendung der Aushärtung der Schichten mittels Mikrowellenbestrahlung dazu, dass das Wasser aus der noch nicht ausgehärteten Mischung der Komponenten A) und B) in die Fasern der Mineralwollschicht einschießt und dabei die Materialmischung mit sich zieht. Dadurch resultiert eine bessere Adhäsion der mittels Härtung gebildeten Schicht der chemisch gebundenen Phosphatkeramik an die benachbarte Mineralwollschicht. Die Eindringtiefe in die Mineralwollschicht beträgt etwa 0,8 bis 1,2 mm und erfüllt, was die Adhäsion beider Schichten angeht, die Anforderungen der Normen DIN EN 1607 und DIN EN 12430.

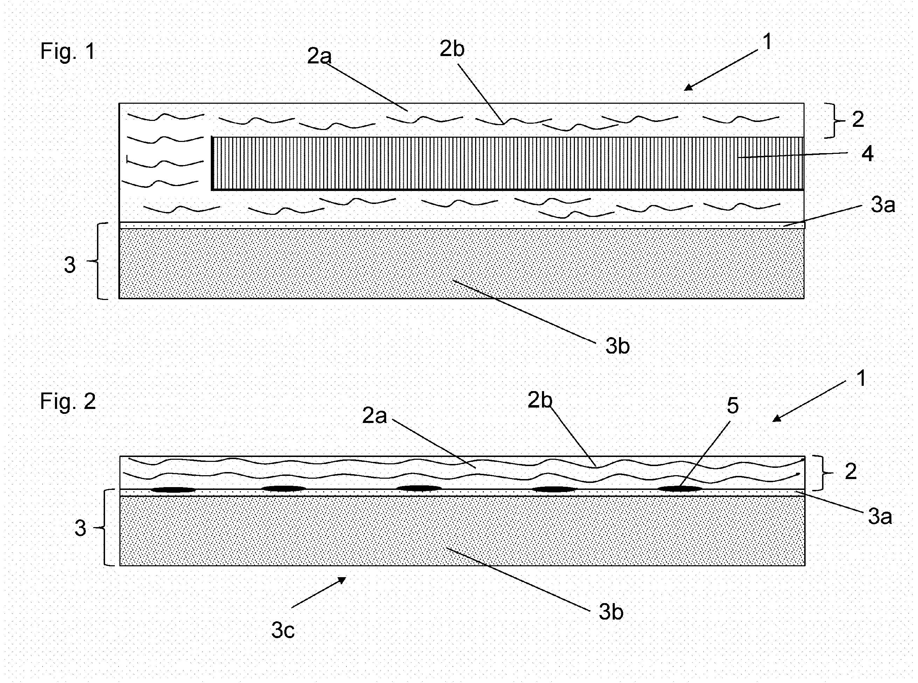

Die Adhäsion zwischen der chemisch gebundenen Phosphatkeramikschicht und der Mineralwollschicht kann dabei so hoch sein, dass bei dem Zugmaschinentest nach DIN EN 1607 die Mineralwollschicht zerreißt und keine Delamination zwischen der Keramikschicht und der Mineralwollschicht stattfindet, was zeigt, dass in diesen Fällen die Kohäsion innerhalb der Mineralwollschicht geringer ist als die Adhäsion an die chemisch gebundene Phosphatkeramikschicht. Dies tritt insbesondere dann auf, wenn keine gezielt eingeführten Zwischenräume zwischen der Phosphatkeramikschicht und der Mineralwollschicht vorhanden sind, die im Brandfall bei hohen Temperaturen zu einer gezielten Delamination führen (siehe Beschreibung weiter unten, auch im Bezug auf Figur 2).

Derartige Kompositmaterialien aus einer Mineralwollschicht und einer darauf angeordneten Phosphatkeramik-Schicht benötigen keine dazwischen befindlichen Klebstoffschichten. Bei herkömmlichen Bauteilen sind häufig Klebstoffschichten zwischen der Phosphatkeramik-Schicht und der Mineralwollschicht vorhanden.

Bei einer weiteren Variante eines beschriebenen Verfahrens zur Herstellung eines Formteils beziehungsweise eines Kompositmaterials wird zusätzlich eine Metallschicht, bevorzugt eine Leichtmetallschicht, verwendet, wobei die Dichte des Metalls unter 3 g/cm³ liegen kann. Diese Metallschicht wird vorzugsweise in die Phosphatkeramikschicht eingebettet, die noch zusätzlich Fasern oder Faserschichten aufweisen kann.

Der Vorteil von Metallschichten, insbesondere Leichtmetallschichten, besteht darin, dass die Festigkeit von beispielsweise Trennwänden oder Halbzeugen und Bauteilen mit derartigen Metallschichten deutlich erhöht wird. Bei der Verwendung von Leichtmetallschichten, die insbesondere auch Leichtmetallwaben, beispielsweise Aluminiumwaben, sein können, werden besonders stabile Bauteile, beispielsweise Trennwände, hergestellt, die zudem sehr leicht sind. Als Leichtmetall kann insbesondere Aluminium verwendet werden.

Anstelle des Handauflegeverfahrens können auch zwei- oder dreidimensionale Formteile unter Verwendung einer der oben genannten Zusammensetzungen dadurch hergestellt werden, dass eine Variante eines hier beschriebenen Verfahrens die folgenden Verfahrensschritte aufweist:
A2) Mischen der Komponenten A) und B),
B2) Einbringen der Mischung der Komponenten A) und B) in die Kavität eines Formwerkzeuges, und
C2) Aushärten der Mischung der Komponenten A) und B) unter Bildung des Formteils mit der chemisch gebundenen Phosphatkeramik.

Ein derartiges Verfahren kann zum Herstellen von Formteilen mittels Pressen oder Spritzgießen eingesetzt werden. Beim Pressen oder Spritzgießen wird eine zumindest zweiteilige, aber auch mehrteilige Form verwendet, die eine Kavität aufweist, in die die Mischung der Komponenten A) und B) eingebracht wird. Die Kavität bildet zusammen mit weiteren Bestandteilen des Formwerkzeugs, beispielsweise konkaven Matrizen, eine Negativform zu der gewünschten Endform des Formteils. Die Materialeinsätze sind häufig aus Edelstahl oder Werkzeugstählen mit PTFE entweder im Sprühverfahren oder in Folienapplikationen. Die Werkzeuge sind bis auf 160 °C beheizbar und der Innendruck in den Formwerkzeugen kann > 10 daN/cm² betragen. Die Formhaltezeit der Mischung in dem Formwerkzeug beträgt in Abhängigkeit von der Struktur der Kavität und der Rezeptur der Mischungen A) und B) bis zu 15 Minuten, bevorzugt etwa drei bis 10 Minuten.

Beschrieben wird auch ein Formteil umfassend eine chemisch gebundene Phosphatkeramik, wobei die Phosphatkeramik zwischen 0,2 Massen% bis 2 Massen% Aluminium und zwischen 0,2 Massen% bis 1,5 Massen% Zink enthält.

Es wurde festgestellt, dass derartige chemisch gebundene Phosphatkeramiken gegenüber herkömmlichen, im Handel erhältlichen Vubonite^{®}-Phosphatkeramiken erniedrigte Werte bei Aluminium, insbesondere aber im Bezug auf Zink, aufweisen. Herkömmliche Vubonite^{®}-Phosphatkeramiken weisen in der Regel über 2,7 Massen% Zn auf.

Die Massenprozentanteile dieser Elemente und auch weiterer Elemente können insbesondere mittels Massenspektrometrie mit induktiv gekoppeltem Plasma (ICP-MS) analysiert werden. Somit ist es möglich, besonders einfach bei ausgehärteten Schichten mit chemisch gebundenen Phosphatkeramiken zwischen hier beschriebenen Phosphatkeramiken und herkömmlichen Phosphatkeramiken zu unterscheiden.

Bevorzugt weisen die hier beschriebenen Phosphatkeramiken zwischen 0,5 Massen% bis 1,4 Massen% Aluminium und zwischen 0,5 Massen% bis 1,2 Massen% Zink auf.

Weiterhin kann das Formteil mit der chemisch gebundenen Phosphatkeramik zwischen 6 bis 18 Massen% Phosphor, bevorzugt zwischen 7 bis 12 Massen% P, wie durch ICP-MS bestimmt, enthalten.

Weiterhin kann das Formteil noch zwischen 0,23 bis 0,35 Massen% Na bestimmt durch ICP-MS aufweisen. Dieses Element kann vor allem durch die Metall-Sulfat-Verbindung sowie - falls vorhanden - das Metallhalogenid in die chemisch gebundene Phosphatkeramik eingebracht werden.

Die Massen%-Anteile von B bestimmt durch ICP-MS können zwischen 0,15 Massen% bis 0,35 Massen% betragen.

Die Phosphatkeramik in dem Formteil kann auch zusätzlich zwischen 16 bis 38 Massen%, bevorzugt 20 bis 35 Massen%, weiter bevorzugt 25 bis 30 Massen%, Siliziumdioxid SiO₂ enthalten. Calciumoxid CaO liegt erfindungsgemäss in den bevorzugten Massen%-Anteilen des SiO₂ vor.

Unterschiedliche Massen% an CaO und SiO₂ können vor allem durch unterschiedliche Bezugsquellen für die Komponente B), insbesondere das Wollastonit, bedingt sein, da die Anteile an diesen beiden Oxiden zwischen unterschiedlichen Abbauregionen variieren.

Da, wie weiter unten beschrieben, die Analyse der Elemente Si und Ca mittels ICP-MS mit einem großen Fehler behaftet ist, können diese Elemente besser mittels Röntgenfluoreszenzanalyse (RFA) in einer oxidischen Matrix bestimmt werden, wobei die Massen%-Anteile der Oxide der entsprechenden Elemente erhalten werden. Die Massen%-Anteile der weiteren Elemente Al, Zn und P, die mittels ICP-MS bestimmt wurden, konnten dagegen durch die RFA bestätigt werden.

Für feuerfeste Phosphatkeramiken, die im Brandfall keine schädlichen halogenhaltigen Emissionen freisetzen, weist die Phosphatkeramik des Formteils weiterhin weniger als 0,001 Massen% eines Halogens, in der Regel Chlor oder Brom, auf und ist bevorzugt komplett halogenfrei.

Weiterhin ist es bevorzugt, dass die Phosphatkeramik auch keine organischen Bestandteile aufweist, wie bereits oben beschrieben.

Aufgrund des beschriebenen Phosphatkeramikmaterials können die erfindungsgemässen Formteile spannungsfrei hergestellt werden, sodass die Oberfläche der Bauteile spannungsfrei ist. Dies bedeutet insbesondere, dass die Oberfläche dieser Formteile keine Haarrisse oder Adhäsionsbrüche aufweist. Weiterhin kann die Phosphatkeramikschicht besonders dünn in einer Dicke von 0,8 bis 1,8 mm, bevorzugt 1 mm bis 1,5 mm, auf Mineralwollschichten aufgebracht werden.

Die erfindungsgemässen Formteile mit den beschriebenen chemisch gebundenen Phosphatkeramiken können weiterhin eine benachbarte Mineralwollschicht aufweisen. Die Anbindung der Mineralwollschicht an die Phosphatkeramik kann dabei durch Benetzung der Mineralwollschicht mit der Phosphatkeramik vorliegen. Insbesondere kann die Eindringtiefe der Phosphatkeramik in die Mineralwollschicht zwischen 0,8 bis 1,2 mm betragen und bezüglich der Anforderungen an die Adhäsion der Norm DIN EN 1607 entsprechen.

Wie bereits oben beschrieben, kann mittels einer Mikrowellentrocknung das Wasser in die Mineralwollschicht einschießen und dadurch eine besonders stabile Anbindung zwischen der Mineralwollschicht und der chemisch gebundenen Phosphatkeramikschicht erzielt werden. Bei herkömmlichen Kompositmaterialien ist häufig eine Klebstoffschicht zwischen der Mineralwollschicht und der Phosphatkeramikschicht vorhanden, die bei diesen hier beschriebenen Phosphatkeramikschichten nicht notwendig ist.

Mittels der hier beschriebenen chemisch gebundenen Phosphatkeramiken lassen sich insbesondere feuerwiderstandsfähige Formteile herstellen, die zumindest der Klasse IMO FTP Code B15 bis A60 im Schiffbau sowie den Normen DIN EN 13501 im Hochbau und den Normen DIN EN 45545 im Schienenfahrzeugbau entsprechen. Diese Formteile können geringe Wärmeleitfähigkeiten bis unter 110 mW/(m·K) aufweisen.

Im Folgenden soll die Erfindung und hier beschriebene Zusammensetzungen anhand von Ausführungsbeispielen und Figuren noch näher erläutert werden.

Die Figuren 1 bis 4 zeigen verschiedene Ausführungsformen von Formteilen beziehungsweise Laminatanordnungen, die Schichten mit der erfindungsgemäßen Phosphatkeramik enthalten, im Querschnitt.

Figur 5 zeigt eine Oberflächenfotografie eines Bauteils aus einer herkömmlichen Phosphatkeramik mit Haarrissen.

Figur 1 zeigt einen Ausschnitt eines Bauteils 1, das eine Schicht 2 aufweist, die das hier beschriebenen chemisch gebundene Phosphatkeramikmaterial 2a aufweist. Dieses Keramikmaterial dient dabei als Matrix für Fasermaterialien 2b. Anstelle von Fasern können auch Fasermatten beziehungsweise Faserschichten in das Phosphatkeramikmaterial als Matrix eingebettet sein. Weiterhin ist noch eine Leichtmetallschicht, beispielsweise eine Aluminiumwabe 4, in die Phosphatkeramikschicht 2 eingebettet. Benachbart zu der Phosphatkeramikschicht 2 ist eine Mineralwollschicht 3 angeordnet, wobei ein kleiner Teilbereich 3a der Mineralwollschicht mit einer Eindringtiefe von typischerweise 0,8 bis 1,2 mm mit der Phosphatkeramik getränkt ist und ein größerer Teilbereich 3b nicht mit der Phosphatkeramik getränkt ist. Dadurch resultiert eine besonders feste Anbindung der Mineralwollschicht 3 an die Phosphatkeramikschicht 2. Derartige Bauteile können als sogenannte Leichtbauteile beispielsweise in Schiffen, Schienenfahrzeugen oder Flugzeugen verbaut werden.

Figur 2 zeigt im Querschnitt ein feuerwiderstandsfähiges Bauteil 1, das ebenfalls wieder eine Schicht 2 mit der erfindungsgemäßen chemisch gebundenen Phosphatkeramik 2a enthält, wobei diese in diesem Fall als Matrix für Faserschichten 2b dient. Durch teilweises Antrocknen der Phosphatkeramikschicht, bevor beispielsweise die Mikrowellenbestrahlung durchgeführt wird, können zwischen der Phosphatkeramikschicht 2 und der darunter befindlichen Mineralwollschicht 3 Zwischenräume 5 geschaffen werden. Im Brandfall kann es bei Temperaturen von mehr als 700°C aufgrund der Zwischenräume besonders einfach zur Delamination der Phosphatschicht von der Mineralwollschicht kommen, so dass sich zwischen beiden Schichten ein Gang bildet, der eine Isolation aus Gas bildet. Dieser Gang kann heiße Brandgase in den Brandraum zurückleiten, so dass im Brandfall die Temperatur der dem Brand abgewandten Seite 3c des Bauteils, nämlich die der Phosphatschicht abgewandten Seite der Mineralwollschicht, nicht unzulässig erhöht wird.

Die Mineralwollschicht 3 weist dabei ähnlich zu Figur 1 wieder einen nicht mit der Phosphatkeramik getränkten größeren Schichtbereich 3b sowie eine kleine, durch eine Eindringtiefe von 0,8 bis 1,2 mm gekennzeichnete Schicht 3a auf, die mit der Phosphatkeramik getränkt ist. Ein größerer Teilbereich 3b der Mineralwollschicht 3 ist nicht mit der Phosphatkeramik getränkt.

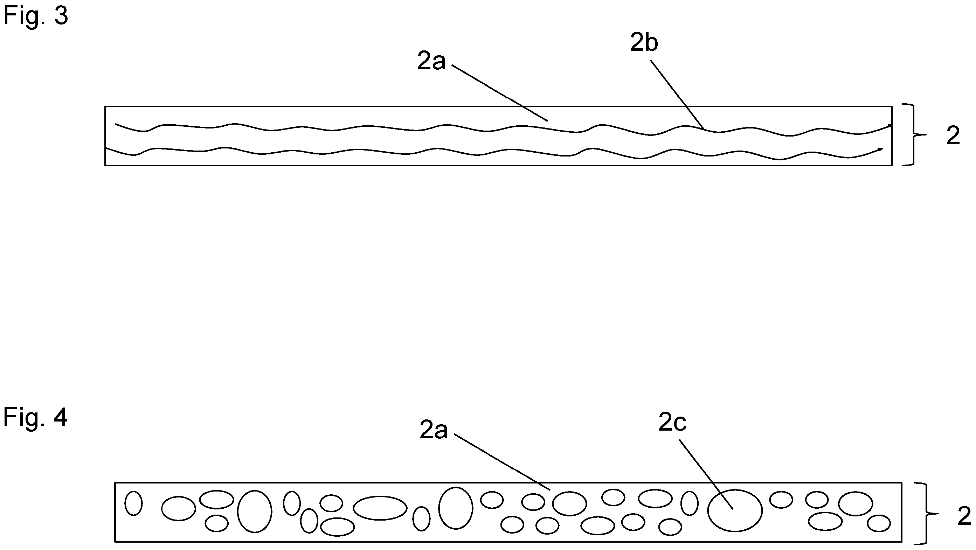

Figur 3 zeigt im Querschnitt eine Variante eines einfachen Formteils, bei der lediglich eine Phosphatkeramik enthaltende Schicht 2 vorhanden ist, wobei in die Phosphatkeramik 2a wiederum Faserschichten 2b eingelagert sind.

Figur 4 zeigt im Querschnitt eine weitere Ausführungsform eines erfindungsgemäßen Formteils, bei der eine Phosphatkeramik enthaltende Schicht 2 vorhanden ist, die mittels Schäumens mit Schäumungsmitteln ausgehärtet wurde. Dabei bilden sich in der Phosphatkeramik-Schicht 2a interne Hohlräume, die Zellen 2c, die die Dichte der Schicht erheblich herabsetzen und so eine große Gewichtsersparnis ermöglichen.

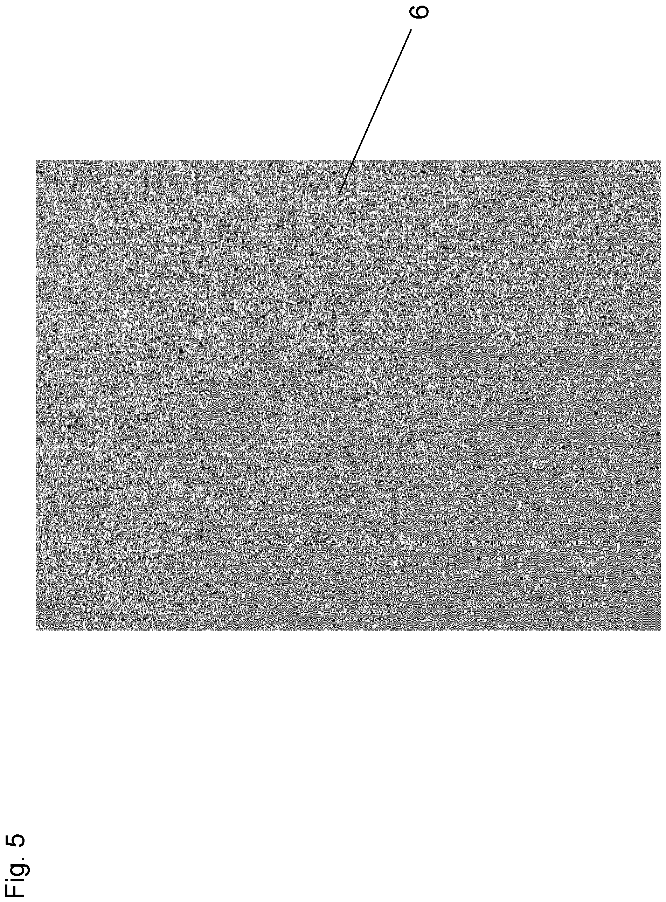

Figur 5 zeigt in der Aufsicht die Oberfläche eines herkömmlichen Formteils, das mit der unter dem Markennamen Vubonite^{®} vertriebenen Phosphatkeramik hergestellt wurde. Deutlich sind dabei Haarrisse 6 zu erkennen, die zeigen, dass die Oberfläche dieses Bauteils nicht spannungsfrei ist. Im Gegensatz dazu weisen die Oberflächen von erfindungsgemäßen Formteilen aufgrund ihrer Spannungsfreiheit keine Haarrisse auf.
1. Ausführungsformen von hier beschriebenen Komponenten A):

Die folgende Tabelle zeigt die Zusammensetzungen von vier verschiedenen Ausführungsformen A1) bis A4) der Komponente A) in Gew% der Komponente A), die zur Herstellung von hier beschriebenen Phosphatkeramiken verwendet werden können (aufgrund der Rundung in der zweiten Nachkommastelle kann die Summe der Zusammensetzungen von 100 Gew% abweichen):

**Tabelle 1:**

| **Komponente** | **H₂0** | **H₃PO₄** | **ZnO** | **Amorphes Al(OH)₃** | **Na₂B₄O₇× 10 H₂O** | **NaBr** | **NaCl** | **Na₂SO₄** |
|---|---|---|---|---|---|---|---|---|
| **A1)** | 26,03 | 63, 61 | 2,19 | 4, 86 | 2,84 | 0,41 | - | 0, 06 |
| **A2)** | 24,40 | 65,36 | 2,16 | 4, 80 | 2, 80 | 0, 41 | - | 0.06 |
| **A3)** | 26,03 | 63, 61 | 2,19 | 4, 86 | 2,84 | - | 0,41 | 0,06 |
| **A4)** | 26, 44 | 63, 61 | 2, 19 | 4,86 | 2,84 | - | - | 0,06 |

Dabei ist besonders die Komponente A4) von Interesse, da diese halogenfrei ist und somit besonders gut zur Herstellung feuerfester halogenfreier Formteile geeignet ist.

Weiterhin kann insbesondere Na₂SO₄ aus allen Komponenten A1) bis A4) weggelassen werden. Dies ist insbesondere dann vorteilhaft, wenn ein geschäumtes Gefüge von feiner Qualität erforderlich ist, das auch gut lackiert werden kann.

Diese Komponenten können dadurch hergestellt werden, dass wässrige Phosphorsäure vorgelegt wird und anschließend die einzelnen Komponenten unter Rühren und ggf. Erhitzen in der wässrigen Phosphorsäure gelöst werden.

### 2. Ablauf einer kontrollierten exothermen Polykondensation beim Aushärten einer Mischung der Komponenten A) und B)

Im Folgenden wurden vier verschiedene Proben 1 bis 4 der Mischungen A) und B) gemischt und auf eine Fläche von 0,0122 m² aufgebracht. Als A-Komponente wurde die in Tabelle 1 beschriebene Komponente A1) und als Komponente B) ß-Wollastonit als Feststoff mit einer Korngröße D₅₀ < 70 µm verwendet. Mischungsverhältnisse sowie weitere Versuchsparameter können der folgenden Tabelle entnommen werden:

**Tabelle 2:**

| | **Probe 1** | | **Probe 2** | | **Probe 3** | | **Probe 4** | |
|---|---|---|---|---|---|---|---|---|
| | A1) | B) | A1) | B) | A1) | B) | A1) | B) |
| **Temperatur** | RT (ca. 21°C) | - | RT (ca. 21°C) | - | RT (ca. 21°C) | - | RT (ca. 21°C) | - |
| **Mischungsverhältnis** | 100 | 80 | 100 | 80 | 100 | 80 | 100 | 80 |
| **Ansatzmenge [g]** | 100 | 80 | 200 | 160 | 400 | 320 | 800 | 640 |
| **l/min.** | ~ 1.000 | | ~ 1.000 | | ~ 1.000 | | ~ 1.000 | |
| **Rührzeit [sek.]** | 180" | | 180'' | | 180" | | 180'' | |

Die Temperatur der Mischungen wurde kontinuierlich mit dem Meßgerät ebro EBI 40 TC-01 gemessen.

Bei allen Proben stieg nach dem Mischen die Temperatur von etwa 25°C bis auf maximal 31°C über einen Zeitraum von etwa maximal 2,5 Std. Somit lief eine kontrollierte exotherme Polykondensation ab, bei der die Temperatur um nicht mehr als 20°C pro m² Oberfläche an gebildeter chemisch gebundener Phosphatkeramik anstieg.

**Tabelle 3:**

| | **Probe 1** | | **Probe 2** | | **Probe 3** | | **Probe 4** | |
|---|---|---|---|---|---|---|---|---|
| Komponenten | A1) | B) | A1) | B) | A1) | B) | A1) | B) |
| Temperatur | RT (ca. 23°C) | - | RT (ca. 23°C) | - | RT (ca. 23°C) | - | RT (ca. 23°C) | - |
| Mischungsverhältnis | 100 | 100 | 100 | 100 | 100 | 100 | 100 | 100 |
| Ansatzmenge [kg] | 1,35 | 1,35 | 1, 90 | 1, 90 | 2,55 | 2, 55 | 3, 75 | 3,75 |
| Rührzeit [sek.] | 180 | | 180 | | 180 | | 180 | |
| Lagenaufbau 1 m² Fläche | 2 Faserschichten | | 3 Faserschichten | | 4 Faserschichten | | 5 Faserschichten | |

An jeder Probe wurden Temperatursensoren an den jeweiligen vier Eckpunkten auf einer Fläche von 0,64 m² positioniert. Bei allen Proben stieg nach dem Mischen die Temperatur von etwa 26 °C bis auf maximal 35 °C über einen Zeitraum von etwa maximal 2,0 bis 3,5 Std. Somit lief eine kontrollierte exotherme Polykondensationsreaktion ab, bei der die Temperatur um nicht mehr als 20°C an gebildeter chemisch gebundener Phosphatkeramik anstieg.

### 3. Elementaranalysen von chemisch gebundenen Phosphatkeramiken:

Im Folgenden werden Elementaranalysen von erfindungsgemäßen Formteilen (Proben 5 bis 9) tabellarisch aufgelistet, die mittels eines Aushärtens einer Mischung der in Tabelle 1 beschriebenen Komponente A4) im Gewichtsverhältnis 100:100 mit ß-Wollastonit als Komponente B) hergestellt wurden. Diese hier beschriebenen Materialien werden in der Elementaranalyse mit Formteilen (Proben 1 bis 4) verglichen, die mit der herkömmlichen Phosphatkeramik Vubonite^{®} hergestellt wurden:

**Tabelle 4 (Anteile der Elemente in Massen%):**

| **Probe** | **Al*** | **Na*** | **CaO**** | **SiO₂**** | **Zn*** | **P*** | **Br*** | **S*** | **B*** |
|---|---|---|---|---|---|---|---|---|---|
| 1 | 1,34 | 0,23 | 25-30 | 25-30 | 2, 87 | 11,22 | < 0,01 | < 0, 01 | 0,21 |
| 2 | 1,12 | 0,23 | " | " | 2, 79 | 10,84 | < 0,01 | < 0, 01 | 0,20 |
| 3 | 1,46 | 0, 31 | " | " | 2, 89 | 10,91 | < 0,01 | < 0, 01 | 0,24 |
| 4 | 1,42 | 0,26 | n.b. | n.b. | 3,22 | 11,22 | < 0, 01 | < 0, 01 | 0,22 |
| 5 | 1,02 | 0,29 | 25-30 | 25-30 | 0,86 | 10,81 | < 0, 01 | < 0,01 | 0, 18 |
| 6 | 0, 89 | 0,29 | " | " | 0, 88 | 10,82 | < 0, 01 | < 0,01 | 0, 18 |
| 7 | 0, 95 | 0,30 | " | " | 0, 87 | 11,29 | < 0,01 | < 0, 01 | 0, 19 |
| 8 | 1,07 | 0,24 | " | " | 0, 90 | 10,65 | < 0,01 | < 0,01 | 0,20 |
| 9 | 1,00 | 0,25 | n.b. | n.b. | 0, 94 | 11,22 | < 0,01 | < 0,01 | 0,22 |

| | | | | | | | | | |
|---|---|---|---|---|---|---|---|---|---|
| n.b. = nicht bestimmt * = Werte mittels ICP-MS bestimmt ** = Werte mittels RFA bestimmt | | | | | | | | | |

Für die Analyse der chemisch gebundenen Phosphatkeramiken wurde dabei das Matrixmaterial der Phosphatkeramik zwischen dem in der Matrix eingebetteten Fasermaterial entfernt und anschließend mit HNO₃/HF aufgeschlossen. Dabei fällt nach kurzer Zeit ein wasserklarer Niederschlag aus, der aller Wahrscheinlichkeit nach gefällte Kieselsäure ist. Die mittels ICP-MS bestimmten Werte für die Elemente Al, Na, Zn und P wurden durch die RFA-Analyse bestätigt, während die Werte für Si und Ca wohl aufgrund des Kieselsäureniederschlags zu gering waren. Aus diesem Grunde wurden die Werte von SiO₂ und CaO in die Tabelle 4 mit aufgenommen, die mittels RFA in einer oxidischen Matrix bestimmt wurden. Die Werte für S und Br liegen für ICP-MS unterhalb der Nachweisgrenze.

Deutlich ist zu erkennen, dass die hier beschriebenen Phosphatkeramiken im Vergleich zu den herkömmlichen Vubonite^{®}-Keramiken weniger Al, insbesondere aber weniger Zn, aufweisen.

Die Erfindung ist nicht durch die Ausführungsbeispiele beschränkt.

## Patentansprüche

1. Formteil, umfassend eine chemisch gebundene Phosphatkeramik, wobei die Phosphatkeramik zwischen 0,2 Massen% bis 2 Massen% Al, bestimmt durch Massenspektrometrie mit induktiv gekoppeltem Plasma, zwischen 0,2 Massen% bis 1,5 Massen% Zn, bestimmt durch Massenspektrometrie mit induktiv gekoppeltem Plasma, und zwischen 20 bis 35 Massen% CaO, bestimmt durch Röntgenfluoreszenzanalyse, enthält.

2. Formteil nach dem vorhergehenden Patentanspruch, wobei die Phosphatkeramik zwischen 0,5 Massen% bis 1,4 Massen% Al und zwischen 0,5 Massen% bis 1,2 Massen% Zn enthält.

3. Formteil nach einem der vorhergehenden Patentansprüche 1 oder 2, wobei die Phosphatkeramik zwischen 6 bis 18 Massen% Phosphor, bevorzugt zwischen 7 bis 12 Massen% P, bestimmt durch Massenspektrometrie mit induktiv gekoppeltem Plasma, enthält.

4. Formteil nach einem der vorhergehenden Patentansprüche 1 bis 3, wobei die Phosphatkeramik zwischen 25 bis 30 Massen% CaO enthält.

5. Formteil nach einem der vorhergehenden Patentansprüche 1 bis 4, wobei die Phosphatkeramik weiterhin zwischen 16 bis 38 Massen%, bevorzugt 20 bis 35 Massen%, weiter bevorzugt 25 bis 30 Massen%, SiO₂, bestimmt durch Röntgenfluoreszenzanalyse, enthält.

6. Formteil nach einem der vorhergehenden Patentansprüche 1 bis 5, wobei die Phosphatkeramik weiterhin weniger als 0,001 Massen% eines Halogens enthält, bevorzugt halogenfrei, ist.

7. Formteil nach einem der vorhergehenden Patentansprüche 1 bis 6 wobei die Phosphatkeramik keine organischen Bestandteile aufweist.

8. Formteil nach einem der vorhergehenden Patentansprüche 1 bis 7, wobei die Oberfläche des Bauteiles spannungsfrei ist.

9. Formteil nach einem der vorhergehenden Patentansprüche 1 bis 8, weiterhin aufweisend zumindest eine Mineralwollschicht, wobei die Mineralwollschicht mit der Phosphatkeramik getränkt ist.

10. Formteil nach einem der vorhergehenden Patentansprüche 1 bis 8, weiterhin aufweisend eine Mineralwollschicht, benachbart zu einer Schicht aufweisend die Phosphatkeramik, wobei die Anbindung der Mineralwollschicht an die Phosphatkeramik durch Benetzung der Mineralwollschicht mit der Phosphatkeramik vorliegt.

11. Formteil nach einem der vorhergehenden Patentansprüche 1 bis 8, wobei die Phosphatkeramik als Schicht in einer Dicke von 0,8 bis 1,8 mm, bevorzugt 1 mm bis 1,5 mm auf eine Mineralwollschicht aufgebracht ist.

12. Formteil nach Anspruch 10, wobei die Eindringtiefe der Phosphatkeramik in die Mineralwollschicht zwischen 0,8 bis 1,2 mm beträgt und bezüglich der Anforderungen an die Adhäsion der Norm DIN EN 1607 entspricht.

13. Formteil nach einem der vorhergehenden Patentansprüche 9 bis 12, wobei zwischen der Mineralwollschicht und der Phosphatkeramik keine Klebstoffschicht notwendig ist.

14. Formteil nach einem der vorhergehenden Patentansprüche 1 bis 8 das feuerwiderstandsfähig ist und zumindest der Klasse IMO FTP Code B15 bis A60 im Schiffbau sowie den Normen DIN EN 13501 im Hochbau und den Normen DIN EN 45545 im Schienenfahrzeugbau entspricht.

## Claims

1. Moulded part comprising a chemically bonded phosphate ceramic, wherein the phosphate ceramic contains between 0.2% by weight and 2% by weight Al, determined by mass spectrometry with inductively coupled plasma, between 0.2% by weight and 1.5% by weight Zn, determined by mass spectrometry with inductively coupled plasma, and between 20 and 35% by weight CaO, determined by X-ray fluorescence analysis.

2. Moulded part according to the preceding claim, wherein the phosphate ceramic contains between 0.5% by weight and 1.4% by weight Al and between 0.5% by weight and 1.2% by weight Zn.

3. Moulded part according to one of the preceding claims 1 or 2, wherein the phosphate ceramic contains between 6 and 18% by weight phosphorus, preferably between 7 and 12% by weight P, determined by mass spectrometry with inductively coupled plasma.

4. Moulded part according to one of the preceding claims 1 to 3, wherein the phosphate ceramic contains between 25 and 30% by weight CaO.

5. Moulded part according to one of the preceding claims 1 to 4, wherein the phosphate ceramic furthermore contains between 16 and 38% by weight, preferably 20 to 35% by weight, further preferably 25 to 30% by weight, SiO₂, determined by X-ray fluorescence analysis.

6. Moulded part according to one of the preceding claims 1 to 5, wherein the phosphate ceramic furthermore contains less than 0.001% by weight of a halogen, preferably is halogen-free.

7. Moulded part according to one of the preceding claims 1 to 6, wherein the phosphate ceramic has no organic constituents.

8. Moulded part according to one of the preceding claims 1 to 7, wherein the surface of the component is stress-free.

9. Moulded part according to one of the preceding claims 1 to 8, furthermore having at least one mineral wool layer, wherein the mineral wool layer is impregnated with the phosphate ceramic.

10. Moulded part according to one of the preceding claims 1 to 8, furthermore having a mineral wool layer, adjacent to a layer containing the phosphate ceramic, wherein the bonding of the mineral wool layer to the phosphate ceramic exists due to wetting of the mineral wool layer with the phosphate ceramic.

11. Moulded part according to one of the preceding claims 1 to 8, wherein the phosphate ceramic is applied to a mineral wool layer as a layer with a thickness of from 0.8 to 1.8 mm, preferably 1 mm to 1.5 mm.

12. Moulded part according to claim 10, wherein the depth of penetration of the phosphate ceramic into the mineral wool layer is between 0.8 and 1.2 mm and corresponds to the standard DIN EN 1607 in terms of the adhesion requirements.

13. Moulded part according to one of the preceding claims 9 to 12, wherein no adhesive layer is necessary between the mineral wool layer and the phosphate ceramic.

14. Moulded part according to one of the preceding claims 1 to 8, which is fire-resistant and corresponds at least to the IMO FTP Code class B15 to A60 in shipbuilding as well as to the standard DIN EN 13501 in building construction and to the standard DIN EN 45545 in railway vehicle manufacturing.

## Revendications

1. Pièce moulée comprenant une céramique phosphatée chimiquement liée, où la céramique phosphatée contient entre 0,2 % en poids et 2 % en poids d'Al, comme déterminé par spectrométrie de masse à plasma à couplage inductif, entre 0,2 % en poids et 1,5 % en poids de Zn, comme déterminé par spectrométrie de masse à plasma à couplage inductif, et entre 20 et 35 % en poids de CaO, comme déterminé par analyse en fluorescence X.

2. Pièce moulée selon la revendication précédente, où la céramique phosphatée contient entre 0,5 % en poids et 1,4 % en poids d'Al et entre 0,5 % en poids et 1,2 % en poids de Zn.

3. Pièce moulée selon l'une des revendications 1 ou 2 précédentes, où la céramique phosphatée contient entre 6 et 18 % en poids de phosphore, préférablement entre 7 et 12 % en poids de P, comme déterminé par spectrométrie de masse à plasma à couplage inductif.

4. Pièce moulée selon l'une des revendications 1 à 3 précédentes, où la céramique phosphatée contient entre 25 % et 30 % en poids de CaO.

5. Pièce moulée selon l'une des revendications 1 à 4 précédentes, où la céramique phosphatée contient en outre entre 16 et 38 % en poids, préférablement de 20 à 35 % en poids, plus préférablement de 25 à 30 % en poids de SiO₂, comme déterminé par analyse en fluorescence X.

6. Pièce moulée selon l'une des revendications 1 à 5 précédentes, où la céramique phosphatée contient en outre moins de 0,001 % en poids d'un halogène et est préférablement sans halogène.

7. Pièce moulée selon l'une des revendications 1 à 6 précédentes, où la céramique phosphatée ne contient aucun constituant organique.

8. Pièce moulée selon l'une des revendications 1 à 7 précédentes, où la surface du composant est exempte de contraintes.

9. Pièce moulée selon l'une des revendications 1 à 8 précédentes présentant en outre au moins une couche de laine minérale, où la couche de laine minérale est imprégnée de la céramique phosphatée.

10. Pièce moulée selon l'une des revendications 1 à 8 précédentes présentant en outre une couche de laine minérale adjacente à une couche contenant la céramique phosphatée, où la liaison de la couche de laine minérale à la céramique phosphatée existe en raison d'un mouillage de la couche de laine minérale avec la céramique phosphatée.

11. Pièce moulée selon l'une des revendications 1 à 8 précédentes, où la céramique phosphatée est appliquée sur une couche de laine minérale sous la forme d'une couche d'une épaisseur qui va de 0,8 à 1,8 mm, préférablement de 1 mm à 1,5 mm.

12. Pièce moulée selon la revendication 10, où la profondeur de pénétration de la céramique phosphatée dans la couche de laine minérale est entre 0,8 et 1,2 mm et satisfait aux exigences en matière d'adhésion de la norme DIN EN 1607.

13. Pièce moulée selon l'une des revendications 9 à 12 précédentes, où aucune couche adhésive n'est nécessaire entre la couche de laine minérale et la céramique phosphatée.

14. Pièce moulée selon l'une des revendications 1 à 8 précédentes qui est résistante au feu et satisfait au moins aux classes B15 à A60 du code FTP de l'OMI concernant la construction navale, ainsi qu'à la norme DIN EN 13501 concernant la construction de bâtiments et la norme DIN EN 45545 concernant la fabrication de véhicules ferroviaires.
